# EUROPEAN PATENT SPECIFICATION

(11) **EP 4 510 961 B1**
(45) Date of publication and mention of the grant of the patent: **20.08.2025**
(21) Application number: 24739198.0
(22) Date of filing: 03.07.2024
(51) Int. Cl.: A61B 34/00, A61B 34/30, A61B 17/00

(54) **ROBOTIC SURGICAL TOOL HOLDERS**
HALTER FÜR ROBOTISCHE CHIRURGISCHE WERKZEUGE
PORTE-OUTILS CHIRURGICAUX ROBOTIQUES

(30) Priority: 04.07.2023 US 202363524911 P
(43) Date of publication of application: 26.02.2025
(60) Divisional application: 25189018.2
(73) Proprietor: LEM SURGICAL AG, 3018 Bern (CH)
(72) Inventor: BAR, Yossi, 3074 Bern (CH); BOZURIC, Antonio, 3097 Liebefeld (CH)
(74) Representative: HGF
(86) International application number: PCT/EP2024/068766
(87) International publication number: WO 2025/008416

(56) References cited:
- WO-A1-2023/284515
- US-A1- 2017 367 782
- US-A1- 2019 142 535
- US-A1- 2022 096 083

## Description

### BACKGROUND

### Field

The disclosed technology relates generally to medical devices and methods for their use. More particularly, the technology relates to surgical robots and tool holders designed to hold different surgical tool types.

A variety of powered and unpowered surgical tools are used in performing robotic surgeries. Many surgical tools used in robotic procedures are similar or identical to those used manually by surgeons in conventional surgical procedures. Such conventional surgical tools may be powered or unpowered and may be held directly or indirectly by a gripper or other tool holder mounted on a robotic surgical arm. For example, as described in commonly owned PCT application no. PCT/IB2023/055047 (published as WO2023/223215) and US Patent Appln. No. 18/631921, a shaft of a conventional ("off-the-shelf") tool may be grasped directly by a gripper or the tool shaft may be inserted through a tubular cannula held by the gripper. In both cases, the robotically manipulated gripper will be used primarily if not exclusively to position the tool relative to the patient, and the tools will be manually operated by the surgeon just as they would be in non-robotic surgeries.

In contrast, other surgical robotic systems both hold and operate surgical tools which are designed to interface with specific tool holders. Such tool holders will have both attachment features and drive features, and the tools can be both positioned and operated by the associated surgical robotic controller.

As the tool-holding interfaces for each type of tool are usually quite different, many robotic surgeries are limited to using tools which are either (a) designed for robotic use but proprietary to the particular robot being used or (b) non-proprietary but not optimized for robotic use. While in many cases it might be desirable to be able to employ a combination of both specialized (proprietary) robotic tools and generic (non-proprietary), that is often difficult or impossible with currently available surgical robotic systems.

For example, some spinal surgical procedures require a range of tools having different purposes and different power and speed requirements. Some applications require high speed and low torque (e.g., drilling), and some applications require low speed and high torque (e.g., screw insertion). Still others require reciprocation, such as sawing.

Even within a particular category of tool, a number of specific tools having different sizes, power and other characteristics will often be required. Given the high complexity and cost of conventional surgical power tools, it is expensive to supply a full range of conventional tools that provide surgeons with a complete selection of size, force and other characteristics.

There is thus a strong need for robotic surgical systems that can use conventional, off-the-shelf surgical tools as well as specialized, proprietary tools with a unique tool-robot interface. In particular, it would be desirable to provide tool holders for use with robotic surgical systems that can interface with a wide variety of surgical tools, including both (a) surgical tools designed to be held, powered, and controlled by the tool holder and robotic surgical system and (b) off-the-shelf surgical tools which are intended primarily for manual use or which for any reason require only that they be held and oriented in a surgical space. It would also be desirable to provide surgical robotic systems intended for use with surgical tools having only mechanical elements which can be sterilized for reuse. At least some if these objectives will be met by the technologies disclosed herein.

### Background Art

Robotic surgical tool holders and interfaces are described in US Patent Nos. 11,832,905; 11,751,954; 10,765,486; 10,813,713;8,479,969: and 8,142,447. Grippers for holding elongate surgical tools and cannulas are described in commonly owned PCT application no. PCT/IB2023/055047 (published as WO2023/223215) and US patent application no. 18/631921. Other commonly owned publications and applications describing surgical robots and tools include PCT application no. PCT/IB2022/052297 (published as WO2022/195460); PCT application no. PCT/IB2022/058986 (published as WO2023/067415); PCT application no. PCT/IB2022/058972 (published as WO2023/118984); PCT application no. PCT/IB2022/058982 (published as WO2023/118985); PCT application no. PCT/IB2022/058978 (published as WO2023/144602); PCT application no. PCT/IB2022/058980 (published as WO2023/152561); PCT application no. PCT/IB2022/058988 (published as WO2023/237922); PCT application no. PCT/IB2023/055439 (published as WO2024/089473); PCT Applications nos.:PCT/IB2023/055662; PCT/EP2024/052338; PCT/IB2023/055663; PCT/EP2024/052338; PCT/IB2023/056911; PCT/EP2024/052353; and US Provisional Application nos.: 63/532,753, 63/568,102, 63/578,395; 63/606,001; 63/609,490; 63/615,076; 63/634161. US 2017/367782 describes an instrument device manipulator (IDM) that is attached to a surgical arm of a robotic system and comprises a surgical tool holder and an outer housing. WO 2023/284515 describes an instrument drive transmission mechanism of a surgical robot and an instrument drive assembly mechanism of a surgical robot.

### SUMMARY

In a first aspect, the disclosed technology provides a robotic surgical tool holder configured to (a) hold but not drive a first type of surgical tool and (b) hold and drive a second type of surgical tool. The robotic surgical tool holder comprises a housing configured to be detachably mounted on a distal end of a surgical robotic arm and has at least one tool-receiving opening configured to removably receive individual surgical tools of either the first type or the second type therethrough. A drive train in the housing includes an input drive member configured to couple to an output drive member on the surgical robotic arm when the housing is mounted on the distal end of the surgical robotic arm. A tool gripper mechanism controllably couplable to the drive train is configured to selectively grasp and release an exterior surface of a surgical tool of the first type of when the surgical tool is positioned in the tool-receiving opening. A tool driver mechanism controllably couplable to the drive train includes an output drive element configured to mechanically engage an input drive element on a surgical tool of the second type when the second type of surgical tool is positioned in the tool-receiving opening or otherwise attached to the tool holder.

While it will often be preferred to stabilize the driven tool within the tool-receiving opening, such positioning is not necessary, and in some instances the driven tool can have one or more operative elements that project from the tool housing into the surgical space without passing through the tool-receiving opening of the tool holder.

The drive train comprises a mechanical arrangement of gears and shafts configured to selectively transmit rotational motion and torque from the output drive member of the surgical robotic arm to each of the tool gripper mechanism and the tool driver mechanism one at a time. In such instances, the robotic surgical tool holder may further comprise a selector mechanism configured to selectively couple the drive train to either the tool gripper mechanism or the tool driver mechanism. In other such instances, the drive train may be configured to automatically couple to either the tool gripper mechanism or the tool driver mechanism.

In some instances, the tool-receiving opening may comprise a cylindrical aperture. In some instances, the tool gripper mechanism may comprise at least one pair of opposed bodies each having a cylindrical peripheral surface with a circumferentially oriented tapered groove formed therein. The tapered grooves are shaped similarly and have partial circular cross-sections with radii that decrease from an initial end of the groove to a terminal end of the groove and wherein the opposed bodies are configured to rotate about their respective axes to orient the tapered grooves to form a gripping surface with a generally continuous circular periphery with (1) a diameter that depends on the rotational positions of the opposed bodies and (2) a center that remains fixed relative to the gripper mechanism regardless of the rotational positions of the opposed bodies. In such instances, the opposed bodies of the tool gripper mechanism may be configured to counterrotate, for example, comprising a shaft having a distal end connected to the tool gripper mechanism and a proximal end driven by the drive train to rotate the shaft to counterrotate the opposed bodies. In specific instances, the drive train includes a vertical shaft having a bevel gear which drives gear wheels connected to each of the opposed bodies.

In some instances, the robotic surgical tool holder further comprises a pair of jaws pivotally attached to the housing, where each jaw may carry one of the opposed bodies of each pair of opposed bodies. For example, the jaws may be configured to move the tapered grooves on the opposed bodies into and out of proximity to facilitate positioning tools therebetween, and the robotic surgical tool holder may further comprise a lever assembly coupled to the shaft and configured to transfer axial translation of the shaft to open and close the jaws.

In some instances, the opposed bodies are configured to control an amount of friction applied to a tool held by the opposed bodies in response to a degree of rotation of the opposed bodies.

In some instances, the output drive element of the tool driver mechanism may be rotatably driven by the drive train and configured to mate with and rotationally drive the input drive element on the second type of surgical tool when an interventional component of said second type of surgical tool is positioned in the tool-receiving opening. For example, the input drive element and the interventional component may be separate, and the housing may have a separate opening for coupling the input drive element to the drive train.

In a second aspect, the disclosed technology provides a robotic surgical system comprising a robotic surgical tool holder as just described in combination with at least one surgical tool of the second type, often with a plurality of surgical tools of the second type.

In a third aspect, the disclosed technology provides an unclaimed method for performing a robotic surgical procedure using at least one of a first type of surgical tool and a second type of surgical tool. The unclaimed method comprises providing a tool holder mounted on a distal end of a surgical robotic arm, where the tool holder includes both (a) a tool gripper mechanism configured to selectively grip and release an exterior surface of the first type of surgical tool and (b) a tool driver mechanism having an output drive element configured to mechanically engage an input drive element on the second type of surgical tool. A surgical tool is selected to be held by the tool holder. If the selected surgical tool is of the first type, the selected surgical tool will be removably gripped in the gripper mechanism of the tool holder. Conversely, if the selected surgical tool is of the second type, the selected surgical tool will be coupled to the tool driver mechanism of the tool holder so that the input drive element of the selected surgical tool couples to the output drive element of the tool driver mechanism.

In some instances, the tool holder may comprise a drive train having an input drive member coupled to an output drive member on the surgical robotic arm and an output drive member configured to selectively couple to either the tool gripper mechanism or the tool driver mechanism. In such instances, the method may further comprise (a) configuring the drive train to couple the output driver member to the tool gripper mechanism and decouple the output driver member from the tool driver mechanism and (b) coupling a surgical tool of the first type to the tool gripper mechanism.

In some instances, such configuring may be effected manually using a mechanical selector coupled to the drive train. Alternatively, such configuring may be effected automatically.

In some instances, the disclosed methods may further comprise (a) configuring the drive train to couple the output driver member to the tool driver mechanism and decouple the output driver member from the tool gripper mechanism and (b) coupling a surgical tool of the second type to the tool driver mechanism. For example, such configuring may be effected manually using a mechanical selector coupled to the drive train. Alternatively, such configuring may be effected automatically.

In some instances, the first type of surgical tools does not require external powering. For example, the first type of surgical tool may be any one of cannulas, independently powered drills, independently powered screw drivers, and independently powered saws which do not require mechanical power from the surgical robot.

In some instances, the second type of surgical tool may comprise any one of drills, screw drivers, and saws which require mechanical power from the surgical robot.

In some instances, performing a robotic surgical procedure may comprise exchanging at least one tool of the first type for at least one tool of the second type or vice versa in the tool holder during the procedure.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:
FIG.1 is a perspective view of a surgical robot having first and second surgical robotic arms which carry tool holders of the disclosed technology and a third surgical robotic arm carrying a navigation camera, in accordance with some embodiments.
FIG. 2 is a perspective view of a tool holder of the disclosed technology aligned with a surgical tool intended to be held but not driven by the tool holder, in accordance with some embodiments.
FIG. 3 is a perspective view of a tool holder of the disclosed technology aligned with a surgical tool intended to be held and driven by the tool holder, in accordance with some embodiments.
FIG. 4 is an isolated view of a gear drive train configured to selectively drive both a gripper mechanism and a tool driver mechanism in a tool holder of the disclosed technology, in accordance with some embodiments.
FIG. 5 is a cross-sectional view of a tool holder of the disclosed technology incorporating the gear drive train of FIG. 4, in accordance with some embodiments.
FIGS. 6A and 6B illustrate a first alternative drive train in accordance with the disclosed technologies, in accordance with some embodiments.
FIGS. 7A, 7B, and 7C illustrate a second alternative drive train in accordance with the disclosed technologies, in accordance with some embodiments.
FIG. 8 is a cross-sectional, schematic illustration of a surgical tool configured to be held and driven by the tool holder of the disclosed technology at a rotationally high speed with low torque, in accordance with some embodiments.
FIG. 9 is a cross-sectional, schematic illustration of a surgical tool configured to be held and rotationally driven by the tool holder of the disclosed technology at low speed with torque, in accordance with some embodiments.
FIG. 10 is a cross-sectional, schematic illustration of a surgical tool configured to be held and reciprocatably driven by the tool holder of the disclosed technology for use with for example surgical saws, in accordance with some embodiments.
FIG. 11 illustrates how two tool holders of the disclosed technology can be used together to coordinate the manipulation of separate tools in a robotic surgical procedure of the disclosed technology, in accordance with some embodiments.
FIG. 12 is a detailed view showing coupling of the two tools of FIG. 11, in accordance with some embodiments.

### DETAILED DESCRIPTION

Unless otherwise defined, all technical terms used herein have the same meaning as commonly understood by one of ordinary skill in the art to which this disclosure belongs.

As used herein, the singular forms "a," "an," and "the" include plural references unless the context clearly dictates otherwise. Any reference to "or" herein is intended to encompass "and/or" unless otherwise stated.

As used herein, the term "about" in some cases refers to an amount that is approximately the stated amount.

As used herein, the term "about" refers to an amount that is near the stated amount by 10%, 5%, or 1%, including increments therein.

As used herein, the term "about" in reference to a percentage refers to an amount that is greater or less the stated percentage by 10%, 5%, or 1%, including increments therein.

As used herein, the phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

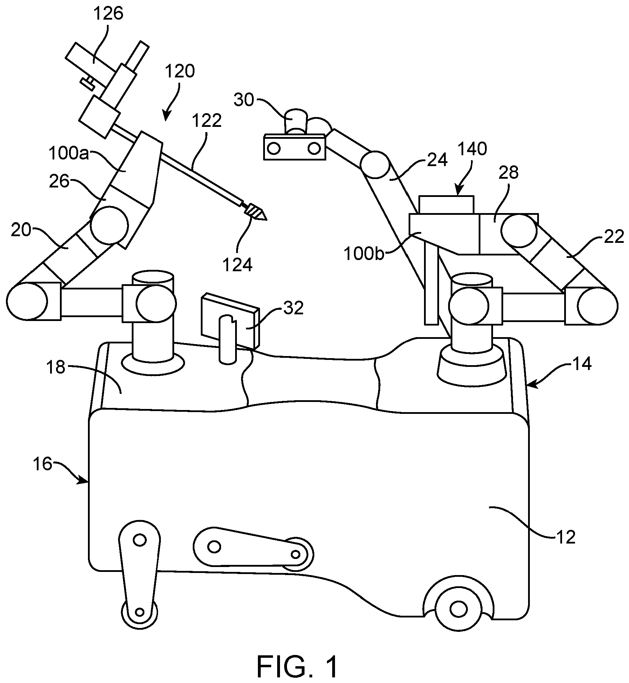

An exemplary robotic surgical system 10 intended particularly for use in the unclaimed methods of the disclosed technology is shown in FIG. 1, in accordance with some embodiments. The robotic surgical system 10 can comprise a chassis 12, typically a single, rigid frame which provides a base or platform for three robotic arms 20, 22 and 24 that are placed relatively far apart on opposite longitudinal ends 14 and 16 of an upper surface 18 of the chassis 12, typically approximately one meter apart, thus allowing for desirable attributes such as reachability, maneuverability, and an ability to apply significant force. In the illustrated embodiment, robotic surgical arms 20 and 22 are on the first end 14 of the chassis 12 and robotic surgical arm 22 is on the second end 16 of the chassis. The chassis can be mobile, e.g., being in the form of a mobile cart as described in commonly owned PCT application no. PCT/IB2022/052297 (published as WO2022/195460). In other embodiments and implementations, the surgical arms 20, 22 and 24 can be mounted on a base or other structure of a surgical table. Placement of the robotic surgical arms on a common, stable platform allows the arms to be moved kinematically or otherwise within a common robotic coordinate system under the control of a surgical robotic controller, typically an on-board controller have a user interface, such as display screen 32.

The single, rigid chassis of the disclosed technology will usually comprise, consist of, or consist essentially of a single mobile cart, as disclosed for example in commonly owned PCT application no. PCT/IB2022/052297 (published as WO2022/195460). In other instances, however, the single, rigid chassis may comprise separate modules, platforms, or components, that are assembled at or near the surgical table, as described for example in commonly owned PCT application no. PCT/EP2024/052353, entitled Integrated Multi-Arm Mobile Surgical Robotic System, filed on January 29, 2024. The only requirement of the single, rigid chassis is that it provide a stable base for all the surgical arms so that they may be accurately and precisely kinematically positioned and tracked by the surgical robotic controller in a single surgical robotic coordinate space.

The chassis 12 of the robotic surgical system 10 can be configured to be temporarily placed under a surgical table (not shown) when performing the robotic surgical procedure, allowing the robotic surgical system 10 to be stored remotely before and after the procedure. The robotic arms 20, 22, and 24 may optionally be configured to be retracted into the chassis 12 of the robotic surgical system, allowing the system to be moved into or out of the surgical field in a compact configuration.

The first and second robotic surgical arms 28 and 28 can have flanges 26 and 28, respectively, mounted at their distal ends. The flanges 26 and 28 each may hold a tool holder 100a and 100b, respectively, which in turn may hold a surgical tool for use in the particular robotic surgical procedure that is being performed. The flanges 26 and 28 can include all electronics and other sensitive system components that cannot be sterilized under harsh conditions, for example, using heat (autoclave) or radiation. The tool holders 100, in contrast, can include only robust mechanical components that can be sterilized and reused in a conventional manner. By providing a surgical drape or other isolation barrier between the tool holder 100 and the flange 26 or 28, the flange can be used in a non-sterile environment and can be reused without needing full sterilization.

The first robotic arm 20 can hold a first tool holder 100a, and the second robotic arm 22 can hold a second tool holder 100b, typically but not necessarily identical to the first tool holder. While the tool holders need not be the same, in order to simplify the present discussion, a single tool holder design will be described hereinafter and be referred by reference number 100.

The structure and use of the tool holders 100 is a central aspect of the disclosed technology and, while the tool holders are particularly suitable for use with mobile and other surgical carts as just described, the disclosed tool holders are suitable for use with most or all surgical robots which include at least one surgical arm for manipulating the tool holders and the tools held by the tool holders in a robotic surgical procedure.

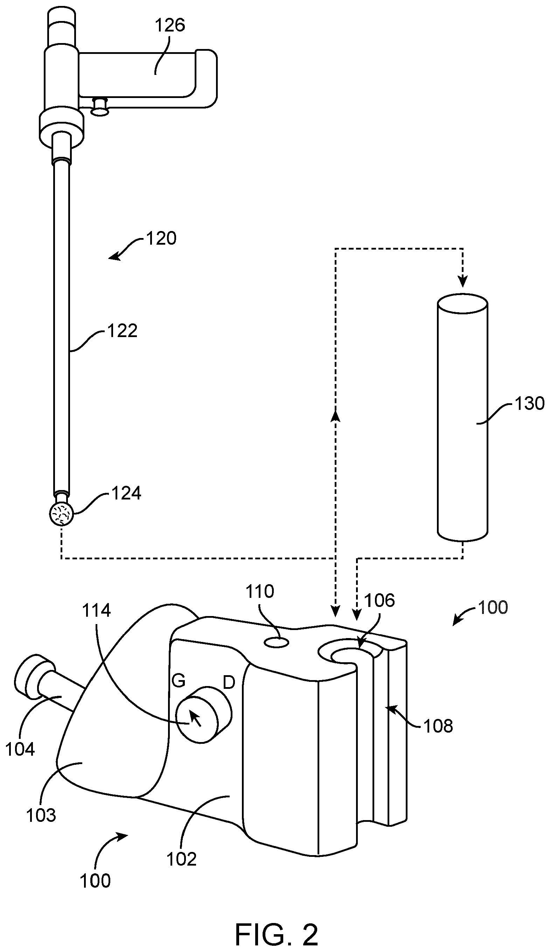

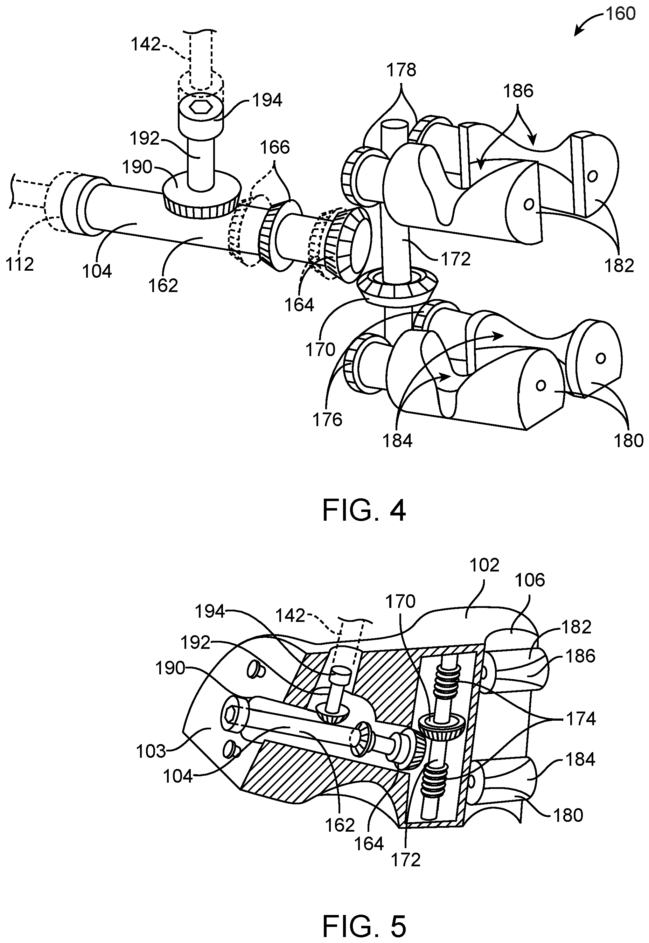

Referring now to FIG. 2, the surgical tool holder 100 comprises a housing 102 having a base 103 and an input drive member 104 on the base, in accordance with some embodiments. The base 103 can be configured to be removably (detachably) attached to an interface, such as flange 26 or 28, at the distal end of robotic surgical arm 20 or 22, as shown in FIG. 1. The input drive member 104 can be configured to be coupled to an output drive member 112 (FIG. 4) disposed on the flange when the tool holder is mounted on the robotic surgical arm. The output drive member 112 can be powered, driven and controlled by the surgical robot, typically but not necessarily being driven by a motor mounted in the flange. A mechanical drive train 160 can be disposed in the housing 102 and configured to selective drive both a gripper mechanism and a tool driver mechanism, as described in more detail with reference to FIGS. 4 and 5. A tool type selector 114 can be located on an exterior of the housing.

A tool-receiving opening 106, typically having a gap 108 along one side thereof, can be formed in an upper surface and near a distal end of the tool holder 100, and a separate tool driver port 110 can also be formed on the upper surface of the tool holder and typically disposed a short distance proximally of the tool receiving opening 108. The tool-receiving opening 106 can be configured to receive both robotically controlled (active) surgical tools and physician-controlled (passive) surgical tool.

The surgical tool holders disclosed herein can be used to hold and manipulate two types or classes of surgical tools, including both (a) OTS "off-the-shelf" surgical tools, such as those suitable for use in non-robotic procedures and typically having a handle, motor, batteries, and the like, which allow them to be used without mechanical or electrical power from a surgical robot or other external source and (b) proprietary and other robotic surgical tools designed specifically to interface with and be mechanically driven by the surgical robot.

As shown in FIG. 2, the surgical tool holder 100 can be used with an OTS "off-the-shelf" surgical tool 120, such as a hand-held grinder, which may be placed directly into the tool-receiving opening 106 of the surgical tool holder 100. In such instances, an internal gripper mechanism (described with reference to FIGS. 4 and 5 below) may be actuated to grasp an outside surface of a component of the tool, such as a cylindrical shaft 122. An initial location of a grinder tip 124 or other active component of the OTS surgical tool can be kinematically registered with the robotic surgical coordinates using conventional techniques, and subsequent positions of the tip 124 can be kinematically tracked based on controlled movements of the supporting robotic surgical arm 26, as shown in FIG. 1. The surgical tool 120 can also be optically tracked using camera 30 or other sensor-based trackers. While positioning of the OTS tool 120 can be controlled by or through the controller 32 of the robotic surgical system 100, a handle 126 of the tool can remain accessible for direct, manual use by the surgeon.

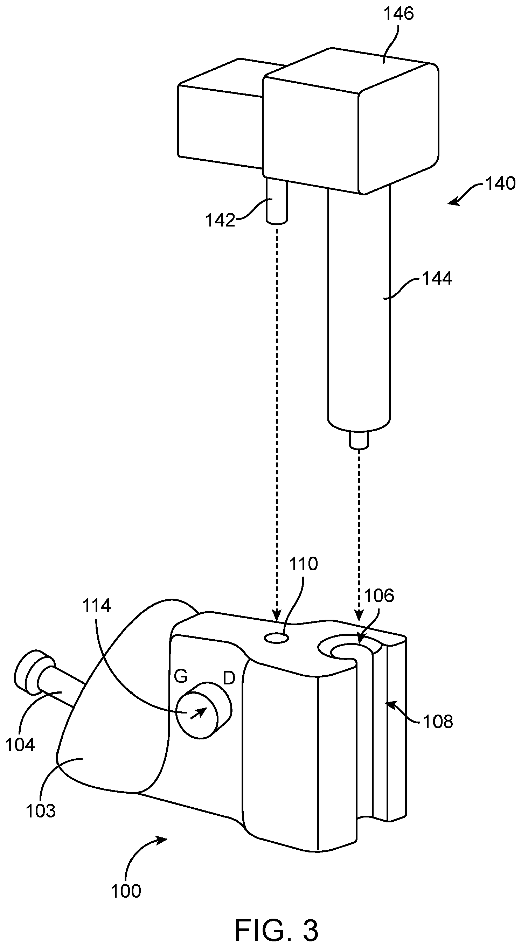

In other instances, a cannula 130 may be introduced directly into the tool-receiving opening 106 of the surgical tool holder 100, typically being used to provide a guide for introducing and exchanging multiple active OTS surgical tools. As indicated by the broken-line paths shown in FIG. 2, for example, the OTS grinder 120 can be introduced through the cannula 130 rather than being paced directly into the gripper mechanism of the tool holder 100. In both instances, the selector switch 114 is turned to the "driven tool" setting (D) to properly connect the drive train 160. As shown in FIG. 3, the driven surgical tool 140 may include a gear housing 146 at its upper end, from which the input drive element 142 extends.

Referring now to FIG. 3, the robotic surgical tool holder 100 can also be used to hold and drive surgical tools 140 of a type which include both an input drive element 142 and a tool shaft 144, in accordance with some embodiments. Such "driven" surgical tools 140 can be constructed to selectively mate with the drive train 160 within the tool holder 100, as described with reference to. FIGS. 4 and 5. In the illustrated embodiment, the tool shaft 144 can be inserted through the tool-receiving opening 106 while the input drive element 142 is simultaneously inserted into tool driver port 110. The selector switch 114 can be turned to the "driven tool" setting (D) to properly connect the drive train 160.

Referring now to FIGS. 4 and 5, the mechanical drive train 160 comprises an assembly of gears and shafts which transmit rotational torque from the output transfer member 112 of the flange 26 or 28 to the input drive member 104 of the tool holder 100, in accordance with some embodiments. The drive train 160 can be located in the tool holder housing 102 but is shown in isolation for simplified presentation. The input drive member 104 can comprise a main drive shaft 162 having a gripper drive gear 164 and tool drive gear 166 thereon. The main drive shaft 162 can be advanced and retracted by the tool-type selector 114 to operate either the gripper function or the tool drive function of the tool holder, as described in more detail below.

As shown in full line in FIG. 4, to operate the gripper function of the tool holder, the main drive shaft 162 can be advanced so that the gripper drive gear 164 engages a bevel drive gear on a vertical drive shaft 172 having upper and lower worm gears 174 (best seen in FIG. 5) which engage and drive worm gears 176 and 178 to rotate the opposed bodies 180 and 182. Rotation of the opposed body pairs 180 and 182 can adjust the diameter of an aperture formed by tapered grooves 184 and 186 to accommodate surgical tools having shafts or other components of different sizes, as described in detail in in commonly owned PCT application no. PCT/IB2023/055047 (published as WO2023/223215) and US patent application no. 18/631921

In order to effect the tool drive function of the tool holder 100, the selector switch can be changed to its D position, as shown in FIG. 3, causing the main drive shaft 162 to retract (move to the left in FIGS. 4 and 5) to both (a) disengage the gripper drive gear 164 from the bevel drive gear 170 as shown in full line in FIG. 4 and to (b) engage the tool drive gear 166 with an drive shaft gear with an output drive shaft 192 having an output coupling member 194 exposed through the tool driver port 110 (FIG. 3). In this way the input drive element 142 can connect to the output coupling member 194 when the driven surgical tool is mounted on the tool holder housing, as illustrated in FIG. 3. The output drive member 112 may be driven by a motor (not illustrated) located in the non-sterile flange 26 or 28, typically a stepper motor.

Referring now to FIG. 5, certain components of the drive train 160 are shown in a cross-section of the tool holder housing 102, in accordance with some embodiments. The worm gears 176 and 178 have been removed from the view to reveal the worm gears 174 that drive the vertical drive shaft 172 to rotate the opposed body pairs 180 and 182 with only one of each pair of opposed bodies be seen. Also, the gripper drive gear 164 is shown beneath the bevel drive gear 170 in FIG. 5 in contrast to in FIG. 4 where the gripper drive gear is shown above the bevel drive gear.

While a specific tool gripping mechanism is described, the term "gripper" and phrase "tool gripper" as used herein and in the claims refers to any mechanical closure device that has a variable aperture for grasping a tool or other surgical object that is to be held and manipulated by the tool gripper. Grippers comprising rotatable opposed bodies can be positioned or adjusted in some way to open and close about a tool or other object positioned therebetween. The opposed bodies can be rotatable (configured to rotate about their respective axes) to orient tapered grooves 184 and 186 on their outer surfaces to form a gripping surface with a generally continuous circular periphery with (1) a diameter that depends on the rotational positions of the opposed bodies and (2) a center that remains fixed relative to the gripper mechanism regardless of the rotational positions of the opposed bodies.

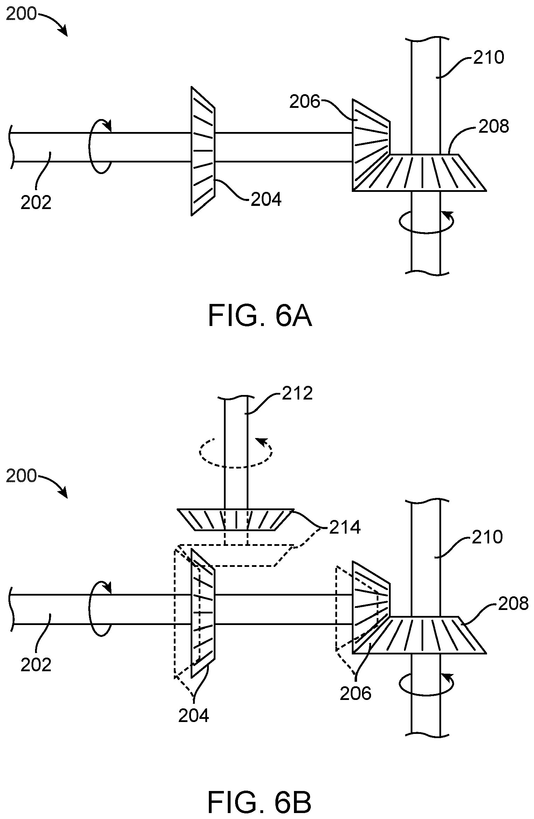

A first alternative drive train assembly 200 is illustrated in FIGS. 6A and 6B, in accordance with some embodiments. While similar to the arrangement shown in FIGS. 4 and 5, the drive train 200 can be configured to drive the gripper so long as no separate driven tool is mounted on the tool holder. A main drive shaft 202 can be rotated in the direction of the arrow by a motor in the flange, as previously described. A beveled tool driver gear 204 can be fixed to rotate with the main drive shaft 202, but no mating gear is provided in the drive train as was the case with the tool holder 100 previously described. A beveled gripper drive gear 206 can also be fixed to rotate with the main drive shaft 202 but can be located at a distal end of the shaft where it mates with a beveled gripper follower gear 208 which drives vertical gripper drive shaft 210. The remainer of the gripper drive train can be identical to that described previously.

When a driven tool is mounted on a tool holder which includes drive train 200, tool drive shaft 212 can enter the tool holder housing. The tool drive shaft 212 can carry a beveled tool drive gear 214 which, when introduced, is out of alignment with beveled tool drive gear 204 on the drive shaft 202. The drive shaft 202 can be spring mounted so that when the beveled peripheries of the drive gears 204 and 214 meet, the drive shaft 212 can be moved to the left as shown in broken line in FIG. 6B. Such engagement concurrently can disengage the gripper drive gear 206 from the gripper follower gear 208, also shown in broken line. Attaching the tool which carries the tool drive shaft 212 and tool drive gear 214 to the tool holder can automatically disengage the gripper drive portion of the drive train 200, thus allowing use of the driven tool without need for the user to manually or otherwise disengage the gripper from the drive train 200 in the tool holder. Similarly, when the driven tool is removed from the tool holder, the gripper drive gear 206 can reengage the gripper follower gear 208 under the spring force of the spring mounting of the main drive shaft 202 (the spring has not been shown to simplify illustration).

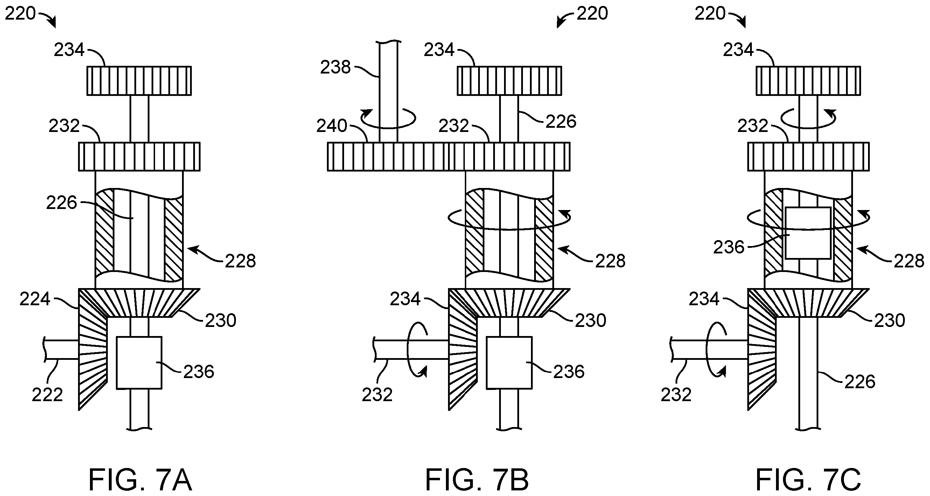

Referring now to FIGS. 7Ato 7C, a second alternative drive train assembly 220 will be described, in accordance with some embodiments. The drive train 220 can comprise a main drive shaft 222 which carries a beveled main drive gear 224 at its distal end. The beveled main drive gear 224 can engage a lower bevel gear 230 which is on a lower portion of a rotating drive structure 228 which also has an upper tool drive gear 232 at its upper end. The rotating drive structure 228 can be formed as a spindle with all portions free to rotate together. The main drive gear 224 can be mounted so that it always engages the lower bevel gear 230 so that the rotating drive structure 228 will always rotate when the main drive shaft 222 is rotated.

The drive train 220 can further include a vertical shaft 226 which extends upwardly through an open interior of the rotating drive structure 228. As shown in FIGS. 7A and 7B, however, the vertical drive shaft 226 may not be coupled to rotating drive structure 228 so that the vertical shaft will not rotate when the rotating drive structure is rotated. The configuration of FIGS. 7A and 7B is intended for driving a tool shaft 238 and tool follower gear 240 of the driven tool, as shown by the arrows in FIG. 7B.

In order to drive the gripper mechanism of the tool holder, a coupling sleeve 236 can be raised to enter the interior of the rotating drive structure 228, as shown in FIG. 7C. The coupling sleeve 236 can be configured to frictionally engage both an interior surface of the rotating drive structure 228 and an exterior surface of the vertical drive shaft 226 so that rotation of the rotating drive structure 228 is transferred to the vertical drive shaft 226 which carries a gripper drive gear at its upper end. The remaining portions of the gripper drive can be similar to the structures described elsewhere herein and commonly owned PCT application no. PCT/IB2023/055047 (published as WO2023/223215) and US application no. 18/631921. While the upper drive gear 232 will still rotate, the driven tool follower gear 240 can be removed so the gear rotation is immaterial. The coupling sleeve 236 can be raised and lowered in a variety of ways, using for example manual linkages, springs, solenoids, and the like.

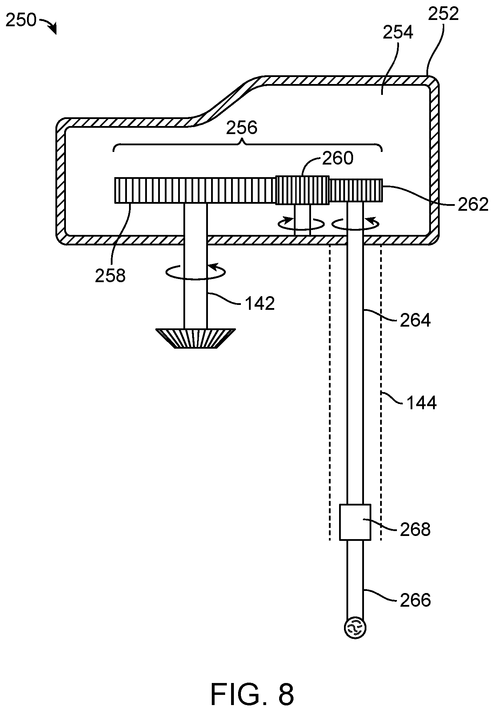

Referring now to FIGS. 8 to 10, several examples of "driven" surgical tools 140, e.g., those designed to be driven by the drive shaft gear 190 and output drive shaft 192 of the tool holders 100, are illustrated, in accordance with some embodiments. The different driven surgical tools 140 can take a variety of forms but will usually share a common external design and identical interface dimensions so that they can be interchangeably mounted on and mechanically coupled to the tool holders of the disclosed technologies. For example, as shown in FIG. 6, a surgical grinder 250 comprises a housing 252 having an interior 254 that holds a drive train 256. The drive train 256 mechanically links the input drive element 142 with the tool shaft 144 as described in FIG. 3. The drive train 256 comprises a drive gear 258 attached to the drive element 142, an idler gear 260, and a follower gear 262 attached to a rotating drive rod 264. By properly selecting the relative diameters of the gears 258, 260, and 262, the rotational speed of the input drive element 142 can be multiplied to achieve high speed (e.g., each having a smaller diameter than the previous gear in the chain), low torque rotation of the rotating rod 264. This is suitable for grinding with the illustrated grinder 266, as well as in a number of applications including drilling, sawing with a rotating blade, polishing, and the like.

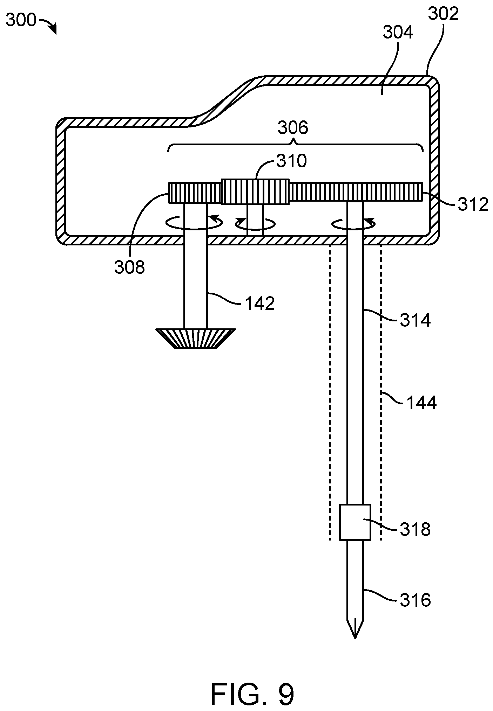

As shown in FIG. 9, a surgical screwdriver 300 comprises a housing 302 having an interior 304 that holds a drive train 306, in accordance with some embodiments. The drive train 306 mechanically links the input drive element 142 with the tool shaft 144 as described in FIG. 3. The drive train 306 comprises a drive gear 308 attached to the drive element 142, an idler gear 310, and a follower gear 312 attached to a rotating drive rod 314. By properly selecting the relative diameters of the gears 308, 310, and 312 (e.g., each having a larger diameter than the previous gear in the chain), the rotational speed of the input drive element 142 can be reduced to achieve low speed, high torque rotation of the rotating rod 314. This is suitable for screwing in pedicle and other surgical screws with the illustrated screwdriver tip 316, as well as in other low speed, high torque applications.

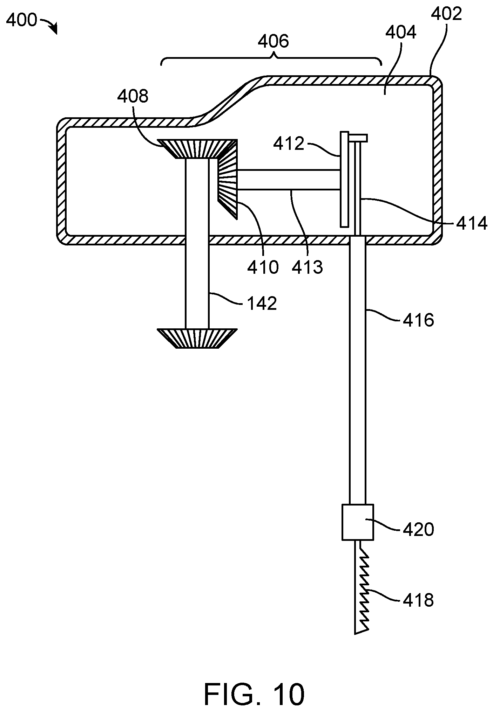

As shown in FIG. 10, a surgical saw 400 comprises a housing 402 having an interior 404 that holds a drive train 406, in accordance with some embodiments. The drive train 406 can mechanically link the input drive element 142 with the tool shaft 144 as described in FIG. 3. The drive train 406 can comprise a beveled drive gear 408 attached to the drive element 142, a beveled follower gear 410, and rotating disc 412. The input drive element 142 can rotate on a vertical axis (as viewed in FIG. 8), and the beveled gears 408 and 410 can cooperate to rotate a connecting shaft 413 on a horizontal axis. The connecting shaft 413, in turn, can rotate the rotating disc 412 in a vertical plane to reciprocate the crank rod 414 in a generally vertical direction. Details of the connection of the rotating disc 412 to the crank rod 414 are not shown, but the connection can be made in a variety of ways known in the art. The crank rod 414 will typically by located in a cover shaft 416 and will reciprocate a saw blade 418 coupled at its distal end by a coupler 420 that allows blade selection before a procedure and replacement of the blade during a procedure.

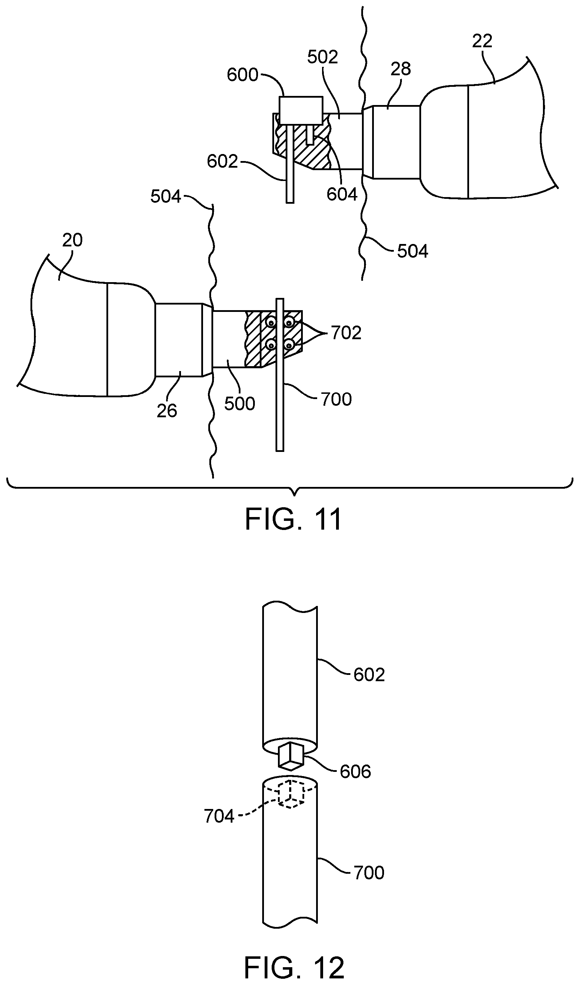

Referring now to FIGS. 11 and 12, two or more tool holders 500 and 502 can be used in combination for performing a robotic surgical procedure, in accordance with some embodiments. The tool holders 500 and 502 can be attached to flanges 26 and 28 which are carried by robotic surgical arms 20 and 22, respectively, as described previously with reference to FIG. 1. Surgical draping 504 can be positioned at the interface between the tools 500 and 502 and the flanges 26 and 28, exposing only the tools to the sterile environment and limiting sterilization for reuse to the tools which include only mechanical components. The flanges 26 and 28, which typically include the motors and electronics needed for operating the tool holders 500 and 502 as well as the surgical tools themselves, can be outside of the sterile field and will not require sterilization for reuse.

Individual surgical tools can be fed to the tool holders in a variety of ways, including both manual and robotically assisted protocols. Manual attachment will rely on the surgeon or a surgical assistant to choose a desired tool from an inventory and manually introduce or attach the tool to the gripper or driver attachment portion of the tool holder. Robotic attachment may utilize dedicated or other mobile carts which carry an inventory of tools and which may incorporate a dedicated arm for selecting tools from the tool inventory and attaching the selected tool to the tool holder, as described in commonly owned PCT application no. PCT/IB2022/058980 (published as WO2023/152561).

The second tool holder 502 can carry a rotational driver 600 having an output drive shaft 602 and an input drive element 604 (similar to the arrangements in both FIGS. 6 and 7), and the output drive shaft 602 can have a coupling feature 606 at a distal tip thereof, as shown in FIG. 10. In this way, the robotic controller 30 can be used to both position the robotic surgical arm 22 in the robotic surgical space and control rotation of the drive shaft 602.

The first tool holder 500 can grip a tool 700 using opposed bodies 702 of an internal gripping mechanism of said first tool holder, generally as described above with reference to FIGS. 3 to 5. The robotic controller 30 can be used to align and engage a coupling feature 704 on the tool 700 with the drive coupling 606 on the output drive shaft 602 of the rotational driver 600 held by the second tool holder 502. Advantageously, the grip of the opposed body pairs 702 can be adjusted by small changes in the rotational orientation of the opposed bodies, allowing the output drive shaft 602 to both rotate and axially position the driven tool 700 relative to the first tool holder 500.

### List of Reference Numbers.

| | |
|---|---|
| 10 | Robotic surgical system |
| 12 | Chassis |
| 14 | First end |
| 16 | Second end |
| 18 | Upper surface |
| 20 | Robotic surgical arm (first) |
| 22 | Robotic surgical arm (second) |
| 24 | Robotic surgical arm (third) |
| 26 | Flange |
| 28 | Flange |
| 30 | Navigation camera |
| 32 | Display/Controller |
| 100 | Surgical tool holder |
| 102 | Housing |
| 103 | Base |
| 104 | Input drive member |
| 106 | Tool-receiving opening |
| 108 | Gap |
| 110 | Tool driver port |
| 112 | Output drive member |
| 114 | Tool type selector |
| 120 | OTS Surgical tool |
| 122 | Shaft |
| 124 | Grinder tip |
| 126 | Handle |
| 130 | Cannula |
| 140 | Driven surgical tool |
| 142 | Input drive element |
| 144 | Tool shaft |
| 160 | Drive train |
| 162 | Main drive shaft |
| 164 | Gripper drive gear |
| 166 | Tool drive gear |
| 170 | Bevel drive gear |
| 172 | Vertical drive shaft |
| 174 | Worm gear |
| 176 | Secondary worm gear |
| 178 | Secondary worm gear |
| 180 | Opposed body pair |
| 182 | Opposed body pair |
| 184 | Tapered groove |
| 186 | Tapered groove |
| 190 | Drive shaft gear |
| 192 | Output drive shaft |
| 194 | Output coupling member |
| 200 | Drive train |
| 202 | Main drive shaft |
| 204 | Tool drive gear |
| 206 | Gripper drive gear |
| 208 | Gripper follower gear |
| 210 | Vertical gripper drive shaft |
| 212 | Tool drive shaft |
| 214 | Tool drive gear |
| 220 | Drive train |
| 222 | Main drive shaft |
| 224 | Main drive gear |
| 226 | Vertical shaft |
| 228 | Rotating drive structure |
| 230 | Lower bevel gear |
| 232 | Upper tool drive gear |
| 234 | Gripper drive gear |
| 236 | Coupling sleeve |
| 238 | Tool shaft |
| 240 | Tool follower gear |
| 250 | Surgical grinder |
| 254 | Interior |
| 256 | Drive train |
| 258 | Drive gear |
| 260 | Idler gear |
| 262 | Follower gear |
| 264 | Rotating drive rod |
| 266 | Grinder tip |
| 268 | Coupler |
| 300 | Surgical screwdriver |
| 302 | Tool housing |
| 304 | Interior |
| 306 | Drive train |
| 308 | Drive gear |
| 310 | Idler gear |
| 312 | Follower gear |
| 314 | Rotating drive rod |
| 316 | Screwdriver tip |
| 318 | Coupler |
| 400 | Surgical saw |
| 402 | Tool housing |
| 404 | Interior |
| 406 | Drive train |
| 408 | Beveled drive gear |
| 410 | Beveled follower gear |
| 412 | Rotating disc |
| 413 | Connecting shaft |
| 414 | Crank rod |
| 416 | Shaft |
| 418 | Saw tip |
| 420 | Coupler |
| 500 | Tool holder |
| 502 | Tool holder |
| 600 | Rotational driver |
| 602 | Output drive shaft |
| 604 | Input drive element |

One of skill in the art will realize that several variations on the disclosed embodiments are possible while staying within the bounds of the disclosed technology. Solely by way of example, different variations in the precise dimensions and components of the mechanical drive train can be used within the scope of the disclosed technology. As another example, further variations in forces applied by the mechanical gears (such as speed and torque in different applications) may be provided while still falling within the scope of the disclosed technology. As a further example, the disclosed technology may take the form of a mechanical gear train for interaction with a force-applying element such as a drill bit or may take the form of a complete power tool, all without departing from the scope of the disclosed technology. All specific embodiments described are representative in nature.

## Claims

1. A robotic surgical tool holder (100) configured to (a) hold a first type of surgical tool and/or (b) hold and drive a second type of surgical tool, said robotic surgical tool holder (100) comprising:
a housing (102) configured to be detachably mounted on a distal end of a surgical robotic arm (20, 22) and having at least one tool-receiving opening (106) configured to removably receive individual surgical tools of either the first type or the second type therethrough;
a drive train (160) in the housing (100) having an input drive member configured to couple to an output drive member on the surgical robotic arm (20, 22) when the housing (102) is mounted on the distal end of the surgical robotic arm (20, 22);
a tool gripper mechanism controllably couplable to the drive train (160), said tool gripper mechanism configured to selectively grasp and release an exterior surface of the first type of surgical tool when said first type of surgical tool is positioned in the tool-receiving opening (106); and
a tool driver mechanism (110) controllably couplable to the drive train (160), said tool driver mechanism having an output drive element (602) configured to mechanically engage an input drive element (604) on the second type of surgical tool when said second type of surgical tool is attached to the tool holder (100);
**characterized in that**
the drive train (160) comprises a mechanical arrangement of gears and shafts configured to selectively transmit rotational motion and torque from the output drive member of the surgical robotic arm (20, 22) to each of the tool gripper mechanism and the tool driver mechanism (110) one at a time.

2. The robotic surgical tool holder of claim 1, further comprising a selector mechanism (114) configured to selectively couple the drive train (160) to either the tool gripper mechanism or the tool driver mechanism.

3. The robotic surgical tool holder of claim 1, wherein the drive train (160) is configured to automatically couple to either the tool gripper mechanism or the tool driver mechanism.

4. The robotic surgical tool holder of one of claims 1-3, wherein the tool-receiving opening comprises a cylindrical aperture.

5. The robotic surgical tool holder of one of claims 1-4, wherein the tool gripper mechanism comprises:
at least one pair of opposed bodies (180, 182) each having a cylindrical peripheral surface with a circumferentially oriented tapered groove (184, 186) formed therein;
wherein the tapered grooves (184, 186) are shaped similarly and have partial circular cross-sections with radii that decrease from an initial end of the groove to a terminal end of the groove and wherein the opposed bodies (180, 182) are configured to rotate about their respective axes to orient the tapered grooves (184, 186) to form a gripping surface with a generally continuous circular periphery with (1) a diameter that depends on the rotational positions of the opposed bodies (180, 182) and (2) a center that remains fixed relative to the gripper mechanism regardless of the rotational positions of the opposed bodies (180, 182).

6. The robotic surgical tool holder of claim 5, wherein the opposed bodies (180, 182) of the tool gripper mechanism are configured to counterrotate.

7. The robotic surgical tool holder of one of claim 5 or 6, further comprising a shaft (222) having a distal end connected to the tool gripper mechanism and a proximal end driven by the drive train (160) to rotate the shaft (222) to counterrotate the opposed bodies (180, 182).

8. The robotic surgical tool holder of one of claims 5-7, wherein the drive train (160) includes a vertical shaft (226) having a bevel gear (230) which drives gear wheels connected to each of the opposed bodies (180, 182).

9. The robotic surgical tool holder of one of claims 5-8, wherein the robotic surgical tool holder further comprises a pair of jaws pivotally attached to the housing (102), optionally wherein each jaw carries one of the opposed bodies (180, 182) of each pair of opposed bodies (180, 182).

10. The robotic surgical tool holder of claim 9, wherein the jaws are configured to move the tapered grooves (184, 186) on the opposed bodies (180, 182) into and out of proximity to facilitate positioning tools therebetween.

11. The robotic surgical tool holder of one of claims 9 or 10, further comprising a lever assembly coupled to the shaft and configured to transfer axial translation of the shaft to open and close the jaws.

12. The robotic surgical tool holder of one of claims 5-11, wherein the opposed bodies (180, 182) are configured to control an amount of friction applied to a tool held by the opposed bodies (180, 182) in response to a degree of rotation of the opposed bodies (180, 182).

13. The robotic surgical tool holder of claims 1-12, wherein the output drive element (602) of the tool driver mechanism is rotatably driven by the drive train (160) and configured to mate with and rotationally drive the input drive element (604) on the second type of surgical tool when an interventional component of said second type of surgical tool is positioned in the tool-receiving opening, wherein the input drive element (604) and the interventional component are separate and the housing (102) has a separate opening for coupling the input drive element (604) to the drive train (160).

14. A robotic surgical system comprising:
a robotic surgical tool holder as in claim 1; and
at least one surgical tool of the second type.

15. The robotic surgical system of claim 14, further comprising a plurality of surgical tools of the second type.

## Patentansprüche

1. Halter (100) für chirurgische Roboterwerkzeuge, der dazu konfiguriert ist, (a) einen ersten Typ eines chirurgischen Werkzeugs zu halten und/oder (b) einen zweiten Typ eines chirurgischen Werkzeugs zu halten und anzutreiben, wobei der Halter (100) für chirurgische Roboterwerkzeuge Folgendes umfasst:
ein Gehäuse (102), das dazu konfiguriert ist, abnehmbar an einem distalen Ende eines chirurgischen Roboterarms (20, 22) montiert zu sein, und mindestens eine Werkzeugaufnahmeöffnung (106) aufweist, die dazu konfiguriert ist, einzelne chirurgische Werkzeuge entweder des ersten Typs oder des zweiten Typs darin entfernbar aufzunehmen;
einen Antriebsstrang (160) in dem Gehäuse (100), der ein Eingangsantriebselement aufweist, das dazu konfiguriert ist, sich an ein Ausgangsantriebselement an dem chirurgischen Roboterarm (20, 22) zu koppeln, wenn das Gehäuse (102) an dem distalen Ende des chirurgischen Roboterarms (20, 22) montiert ist;
einen Werkzeuggreifmechanismus, der steuerbar an den Antriebsstrang (160) koppelbar ist, wobei der Werkzeuggreifmechanismus dazu konfiguriert ist, eine äußere Oberfläche des ersten Typs eines chirurgischen Werkzeugs selektiv zu ergreifen und freizugeben, wenn der erste Typ eines chirurgischen Werkzeugs in der Werkzeugaufnahmeöffnung (106) positioniert ist; und
einen Werkzeugantriebsmechanismus (110), der steuerbar an den Antriebsstrang (160) koppelbar ist, wobei der Werkzeugantriebsmechanismus ein Ausgangsantriebsteil (602) aufweist, das dazu konfiguriert ist, ein Eingangsantriebsteil (604) an dem zweiten Typ eines chirurgischen Werkzeugs mechanisch in Eingriff zu nehmen, wenn der zweite Typ eines chirurgischen Werkzeugs an dem Werkzeughalter (100) angebracht ist;
**dadurch gekennzeichnet, dass**
der Antriebsstrang (160) eine mechanische Anordnung von Zahnrädern und Wellen umfasst, die dazu konfiguriert ist, selektiv eine Drehbewegung und ein Drehmoment von dem Ausgangsantriebselement des chirurgischen Roboterarms (20, 22) einzeln auf jeden von dem Werkzeuggreifmechanismus und dem Werkzeugantriebsmechanismus (110) zu übertragen.

2. Halter für chirurgische Roboterwerkzeuge nach Anspruch 1, ferner umfassend einen Auswahlmechanismus (114), der dazu konfiguriert ist, den Antriebsstrang (160) selektiv entweder an den Werkzeuggreifmechanismus oder den Werkzeugantriebsmechanismus zu koppeln.

3. Halter für chirurgische Roboterwerkzeuge nach Anspruch 1, wobei der Antriebsstrang (160) dazu konfiguriert ist, sich automatisch entweder an den Werkzeuggreifmechanismus oder den Werkzeugantriebsmechanismus zu koppeln.

4. Halter für chirurgische Roboterwerkzeuge nach einem der Ansprüche 1-3, wobei die Werkzeugaufnahmeöffnung ein zylindrisches Loch umfasst.

5. Halter für chirurgische Roboterwerkzeuge nach einem der Ansprüche 1-4, wobei der Werkzeuggreifmechanismus Folgendes umfasst:
mindestens ein Paar gegenüberliegender Körper (180, 182), die jeweils eine zylindrische Randoberfläche mit einer darin ausgebildeten, in Umfangsrichtung ausgerichteten, sich verjüngenden Nut (184, 186) aufweisen;
wobei die sich verjüngenden Nuten (184, 186) ähnlich geformt sind und teilweise kreisförmige Querschnitte mit Radien aufweisen, die von einem Anfangsende der Nut zu einem abschließenden Ende der Nut abnehmen, und wobei die gegenüberliegenden Körper (180, 182) dazu konfiguriert sind, um sich um ihre jeweiligen Achsen zu drehen, um die sich verjüngenden Nuten (184, 186) so auszurichten, dass sie eine Greifoberfläche mit einem im Allgemeinen kontinuierlichen kreisförmigen Umfang mit (1) einem Durchmesser, der von den Drehpositionen der gegenüberliegenden Körper (180, 182) abhängt, und (2) einem Zentrum, das relativ zu dem Greifmechanismus unabhängig von den Drehpositionen der gegenüberliegenden Körper (180, 182) feststehend bleibt, zu bilden.

6. Halter für chirurgische Roboterwerkzeuge nach Anspruch 5, wobei die gegenüberliegenden Körper (180, 182) des Werkzeuggreifmechanismus dazu konfiguriert sind, sich gegenläufig zu drehen.

7. Halter für chirurgische Roboterwerkzeuge nach einem der Ansprüche 5 oder 6, ferner umfassend eine Welle (222), die ein distales Ende, das mit dem Werkzeuggreifmechanismus verbunden ist, und ein proximales Ende aufweist, das durch den Antriebsstrang (160) angetrieben wird, um die Welle (222) so zu drehen, dass sie die gegenüberliegenden Körper (180, 182) gegenläufig dreht.

8. Halter für chirurgische Roboterwerkzeuge nach einem der Ansprüche 5-7, wobei der Antriebsstrang (160) eine vertikale Welle (226) mit einem Kegelrad (230) enthält, das Zahnräder antreibt, die mit jedem der gegenüberliegenden Körper (180, 182) verbunden sind.

9. Halter für chirurgische Roboterwerkzeuge nach einem der Ansprüche 5-8, wobei der Halter für chirurgische Roboterwerkzeuge ferner ein Paar Backen umfasst, die schwenkbar an dem Gehäuse (102) angebracht sind, wobei optional jede Backe einen der gegenüberliegenden Körper (180, 182) jedes Paars gegenüberliegender Körper (180, 182) trägt.

10. Halter für chirurgische Roboterwerkzeuge nach Anspruch 9, wobei die Backen dazu konfiguriert sind, die sich verjüngenden Nuten (184, 186) an den gegenüberliegenden Körpern (180, 182) in die und aus der Nähe zu bewegen, um ein Positionieren von Werkzeugen dazwischen zu erleichtern.

11. Halter für chirurgische Roboterwerkzeuge nach einem der Ansprüche 9 oder 10, ferner umfassend eine Hebelbaugruppe, die an die Welle gekoppelt und dazu konfiguriert ist, eine axiale Verschiebung der Welle zu übertragen, um die Backen zu öffnen und zu schließen.

12. Halter für chirurgische Roboterwerkzeuge nach einem der Ansprüche 5-11, wobei die gegenüberliegenden Körper (180, 182) dazu konfiguriert sind, als Reaktion auf einen Grad der Drehung der gegenüberliegenden Körper (180, 182) eine Stärke einer Reibung zu steuern, die auf ein Werkzeug aufgebracht wird, das durch die gegenüberliegenden Körper (180, 182) gehalten wird.

13. Halter für chirurgische Roboterwerkzeuge nach den Ansprüchen 1-12, wobei das Ausgangsantriebstteil (602) des Werkzeugantriebsmechanismus durch den Antriebsstrang (160) drehbar angetrieben wird und dazu konfiguriert ist, mit dem Eingangsantriebsteil (604) an dem zweiten Typ eines chirurgischen Werkzeugs zusammenzupassen und es drehend anzutreiben, wenn eine Eingriffskomponente des zweiten Typs eines chirurgischen Werkzeugs in der Werkzeugaufnahmeöffnung positioniert ist, wobei das Eingangsantriebsteil (604) und die Eingriffskomponente getrennt sind und das Gehäuse (102) eine getrennte Öffnung zum Koppeln des Eingangsantriebsteils (604) an den Antriebsstrang (160) aufweist.

14. Chirurgisches Robotersystem, umfassend:
einen Halter für chirurgische Roboterwerkzeuge wie in Anspruch 1; und
mindestens ein chirurgisches Werkzeug des zweiten Typs.

15. Chirurgisches Robotersystem nach Anspruch 14, ferner umfassend eine Vielzahl von chirurgischen Werkzeugen des zweiten Typs.

## Revendications

1. Porte-outil chirurgical robotique (100) conçu pour (a) maintenir un premier type d'outil chirurgical et/ou (b) maintenir et entraîner un second type d'outil chirurgical, ledit porte-outil chirurgical robotique (100) comprenant :
un boîtier (102) conçu pour être monté de manière amovible sur une extrémité distale d'un bras robotique chirurgical (20, 22) et comportant au moins une ouverture de réception d'outil (106) conçue pour recevoir de manière amovible des outils chirurgicaux individuels soit du premier type soit du second type en son travers ;
une chaîne cinématique (160) dans le boîtier (100) comportant un élément d'entraînement d'entrée conçu pour s'accoupler à un élément d'entraînement de sortie sur le bras robotique chirurgical (20, 22) lorsque le boîtier (102) est monté sur l'extrémité distale du bras robotique chirurgical (20, 22) ;
un mécanisme de préhension d'outil pouvant être accouplé de manière commandée à la chaîne cinématique (160), ledit mécanisme de préhension d'outil étant conçu pour saisir et libérer de manière sélective une surface externe du premier type d'outil chirurgical lorsque ledit premier type d'outil chirurgical est positionné dans l'ouverture de réception d'outil (106) ; et
un mécanisme d'entraînement d'outil (110) pouvant être accouplé de manière commandée à la chaîne cinématique (160), ledit mécanisme d'entraînement d'outil comportant un élément d'entraînement de sortie (602) conçu pour mettre en prise mécanique un élément d'entraînement d'entrée (604) sur le second type d'outil chirurgical lorsque ledit second type d'outil chirurgical est fixé au porte-outil (100) ;
**caractérisé en ce que** la chaîne cinématique (160) comprend un agencement mécanique d'engrenages et d'arbres conçu pour transmettre de manière sélective un mouvement de rotation et un couple de l'élément d'entraînement de sortie du bras robotique chirurgical (20, 22) à chacun du mécanisme de préhension d'outil et du mécanisme d'entraînement d'outil (110) l'un après l'autre.

2. Porte-outil chirurgical robotique selon la revendication 1, comprenant en outre un mécanisme de sélection (114) conçu pour coupler de manière sélective la chaîne cinématique (160) soit au mécanisme de préhension d'outil soit au mécanisme d'entraînement d'outil.

3. Porte-outil chirurgical robotique selon la revendication 1, ladite chaîne cinématique (160) étant conçue pour s'accoupler automatiquement soit au mécanisme de préhension d'outil soit au mécanisme d'entraînement d'outil.

4. Porte-outil chirurgical robotique selon l'une quelconque des revendications 1 à 3, ladite ouverture de réception d'outil comprenant une ouverture cylindrique.

5. Porte-outil chirurgical robotique selon l'une quelconque des revendications 1 à 4, ledit mécanisme de préhension d'outil comprenant :
au moins une paire de corps opposés (180, 182) comportant chacun une surface périphérique cylindrique dans laquelle est formée une rainure conique orientée de manière circonférentielle (184, 186) ;
lesdites rainures coniques (184, 186) présentant une forme similaire et comportant des sections transversales circulaires partielles avec des rayons qui diminuent d'une extrémité initiale de la rainure à une extrémité terminale de la rainure et lesdits corps opposés (180, 182) étant conçus pour tourner autour de leurs axes respectifs de manière à orienter les rainures coniques (184, 186) afin de former une surface de préhension avec une périphérie circulaire généralement continue avec (1) un diamètre qui dépend des positions de rotation des corps opposés (180, 182) et (2) un centre qui reste fixe par rapport au mécanisme de préhension indépendamment des positions de rotation des corps opposés (180, 182).

6. Porte-outil chirurgical robotique selon la revendication 5, lesdits corps opposés (180, 182) du mécanisme de préhension d'outil étant conçus pour tourner en sens inverse.

7. Porte-outil chirurgical robotique selon l'une quelconque des revendications 5 ou 6, comprenant en outre un arbre (222) comportant une extrémité distale reliée au mécanisme de préhension d'outil et une extrémité proximale entraînée par la chaîne cinématique (160) de manière à faire tourner l'arbre (222) afin de faire tourner les corps opposés (180, 182) en sens inverse.

8. Porte-outil chirurgical robotique selon l'une quelconque des revendications 5 à 7, ladite chaîne cinématique (160) comprenant un arbre vertical (226) comportant un engrenage conique (230) qui entraîne des roues dentées reliées à chacun des corps opposés (180, 182).

9. Porte-outil chirurgical robotique selon l'une quelconque des revendications 5 à 8, ledit porte-outil chirurgical robotique comprenant en outre une paire de mâchoires fixées de manière pivotante au boîtier (102), chaque mâchoire portant éventuellement l'un des corps opposés (180, 182) de chaque paire de corps opposés (180, 182).

10. Porte-outil chirurgical robotique selon la revendication 9, lesdites mâchoires étant conçues pour déplacer les rainures coniques (184, 186) sur les corps opposés (180, 182) dans et hors de la proximité de manière à faciliter le positionnement d'outils entre eux.

11. Porte-outil chirurgical robotique selon l'une quelconque des revendications 9 ou 10, comprenant en outre un ensemble de levier couplé à l'arbre et conçu pour transférer une translation axiale de l'arbre de manière à ouvrir et fermer les mâchoires.

12. Porte-outil chirurgical robotique selon l'une quelconque des revendications 5 à 11, lesdits corps opposés (180, 182) étant conçus pour commander une quantité de frottement appliquée sur un outil tenu par les corps opposés (180, 182) en réponse à un degré de rotation des corps opposés (180, 182).

13. Porte-outil chirurgical robotique selon les revendications 1 à 12, ledit élément d'entraînement de sortie (602) du mécanisme d'entraînement d'outil étant entraîné en rotation par la chaîne cinématique (160) et conçu pour s'accoupler avec et entraîner en rotation l'élément d'entraînement d'entrée (604) sur le second type d'outil chirurgical lorsqu'un composant d'intervention dudit second type d'outil chirurgical est positionné dans l'ouverture de réception d'outil, ledit élément d'entraînement d'entrée (604) et ledit composant d'intervention étant séparés et ledit boîtier (102) comportant une ouverture distincte destinée à accoupler l'élément d'entraînement d'entrée (604) à la chaîne cinématique (160).

14. Système chirurgical robotique comprenant :
un porte-outil chirurgical robotique selon la revendication 1 ; et
au moins un outil chirurgical du second type.

15. Système chirurgical robotique selon la revendication 14, comprenant en outre une pluralité d'outils chirurgicaux du second type.
